(19) Europäisches Patentamt
European Patent Office
Office européen des brevets

(11) **EP 4 518 909 B1**

(12) **EUROPEAN PATENT SPECIFICATION**

(45) Date of publication and mention of the grant of the patent:
**23.09.2026 Bulletin 2026/39**

(21) Application number: **23724770.5**

(22) Date of filing: **04.05.2023**

(51) International Patent Classification (IPC):
***A61K 49/06*** *(2006.01)*

(52) Cooperative Patent Classification (CPC):
**A61K 49/06**

(86) International application number:
**PCT/EP2023/061741**

(87) International publication number:
**WO 2023/213916 (09.11.2023 Gazette 2023/45)**

(54) **MRI COMPOSITION AND ITS USE IN NONFASTING SUBJECTS**

MRT-ZUSAMMENSETZUNG UND IHRE VERWENDUNG BEI NICHT NÜCHTERNEN PROBANDEN

COMPOSITION D'IRM ET SON UTILISATION CHEZ DES SUJETS AVEC UNE ALIMENTATION SANS RESTRICTION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.05.2022 EP 22171769**

(43) Date of publication of application:
**12.03.2025 Bulletin 2025/11**

(73) Proprietor: **Ascelia Pharma AB**
**215 32 Malmö (SE)**

(72) Inventors:
- **OLAFSSON CORFITZEN, Magnus**
  **215 32 Malmö (SE)**
- **NORLIN, Andreas**
  **215 32 Malmö (SE)**

(74) Representative: **Høiberg P/S**
**Adelgade 12**
**1304 Copenhagen K (DK)**

(56) References cited:

- **CHABANOVA E ET AL: "Imaging Liver Metastases with a New Oral Manganese-Based Contrast Agent", ACADEMIC RADIOLOGY, ELSEVIER, AMSTERDAM, NL, vol. 13, no. 7, 1 July 2006 (2006-07-01), pages 827 - 832, XP028012651, ISSN: 1076-6332, [retrieved on 20060701], DOI: 10.1016/J.ACRA.2006.03.013**
- **ANONYMOUS: "Study Indicates Oral MR Imaging Agent Well Tolerated with Food", 19 October 2021 (2021-10-19), XP055967176, Retrieved from the Internet <URL:https://www.appliedradiology.com/communities/MR-Community/study-indicates-oral-mr-imaging-agent-well-tolerated-with-food> [retrieved on 20221003]**
- **LEANDER P ET AL: "Orally Administered Manganese With and Without Ascorbic Acid as a Liver-Specific Contrast Agent and Bowel Marker for Magnetic Resonance Imaging : Phase I Clinical Trial Assessing Efficacy and Safety", INVESTIGATIVE RADIOLOGY, vol. 45, no. 9, 1 September 2010 (2010-09-01), US, pages 559 - 564, XP055967209, ISSN: 0020-9996, DOI: 10.1097/RLI.0b013e3181e960ab**
- **ANONYMOUS: "Press Release: Ascelia Pharma's Food Effect Study shows that Orviglance image enhancement of the liver is not reduced by light meal", 10 May 2022 (2022-05-10), XP055967197, Retrieved from the Internet <URL:https://www.ascelia.com/press-releases/> [retrieved on 20221003]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 518 909 B1

- **ANONYMOUS: "Press Release: Ascelia Pharma presents results of Orviglance Food Effect Study at RSNA 2022 showing strong liver enhancement both with light meal and in fasting condition", 30 November 2022 (2022-11-30), XP093063650, Retrieved from the Internet <URL:https://www.ascelia.com/mfn_news/ascelia-pharma-presents-results-of-orviglance-food-effect-study-at-rsna-2022-showing-strong-liver-enhancement-both-with-light-meal-and-in-fasting-condition/> [retrieved on 20230713]**

## Description

### Technical field

The present invention relates to an oral Magnetic Resonance Imaging (MRI) composition and its use in diagnosis in vivo of liver metastases and/or primary liver cancer in subjects having consumed a predefined meal.

### Background

Orviglance is an oral liver imaging drug (i.e. a liver specific contrast agent) for detection and visualization of focal liver lesions, using Magnetic Resonance Imaging ("MRI") in subjects where use of the current gold standard gadolinium-based contrast agents ("GBCAs") may be medically inadvisable or cannot be administered. MRI is a medical imaging technique used in radiology to form pictures of the anatomy and the physiological processes of the body. MRI scanners use strong magnetic fields, magnetic field gradients, and radio waves to generate images of the organs in the body.

Chabanova et al (Academic Radiology, Vol 13, No 7, 2006) disclose an MRI composition comprising manganese chloride tetrahydrate, alanine and vitamin D (like Orviglance) for use in the diagnosis of liver metastases.

A study by Leander et al (Investigative Radiology, 45, 2010) has previously shown that administration of an oral manganese based MRI composition to a nonfasting subject followed by MRI led to very low signal enhancement of the liver and the subject was excluded from further efficacy analyses.

Hence, fasting prior to administration of Orviglance as well as other contrast agents is routine procedure to ensure that the imaging quality is not affected by interactions with the various food components impacting absorption of the contrast agents and/or directly affecting the imaging properties of manganese (II) ions or complexes thereof.

### Summary

The invention is defined by the claims. Any subject matter falling outside the scope of the claims is provided for information purposes only.

The present inventors have confirmed that subjects' consumption of a full meal prior to administration of Orviglance results in a significantly inferior image quality than observed for fasting subjects. Long periods of fasting (> 8h) are generally undesirable and may limit the willingness to use Orviglance, and for some subjects, fasting for prolonged periods of time can impose a health risk or significant inconvenience.

Surprisingly, the present inventors have now demonstrated that a meal with a calorie content of a predefined value or below can be consumed prior to administration of Orviglance to obtain an imaging quality which is at least as high as obtained for fasting subjects. These findings support the diagnostic potential of Orviglance, in particular for subjects where fasting is significantly inconvenient or imposes a health risk.

In a first aspect, an MRI composition is provided comprising, separately or together, a physiologically acceptable manganese (II) compound, a proteinogenic amino acid and/or a vitamin D, and one or more water-soluble excipients; wherein the MRI composition is for use in the diagnosis in vivo of liver metastases and/or primary liver cancer in a subject; wherein the subject has consumed a meal from 0 to 8 hours prior to or up to about 2 hours subsequent to administration of the MRI composition and wherein the calorie content of the meal is about a predefined value or below.

In a second aspect, an MRI composition is provided comprising, separately or together, a physiologically acceptable manganese (II) compound, a proteinogenic amino acid and/or a vitamin D, and one or more water-soluble excipients; wherein the MRI composition is for use in the diagnosis in vivo of liver metastases and/or primary liver cancer in a subject; wherein the subject has consumed a meal from 0 to 8 hours prior to or up to about 2 hours subsequent to administration of the MRI composition and wherein the calorie content of the meal is about 500 kcal or below.

In a third aspect, a method for Magnetic Resonance Imaging (MRI) of a subject is provided comprising, administering a composition comprising separately or together, a physiologically acceptable manganese (II) compound, a proteinogenic amino acid and/or a vitamin D, and one or more water-soluble excipients to the subject; and performing MRI on said subject; wherein the subject has consumed a meal from 0 to 8 hours prior to or up to about 2 hours subsequent to administration of the MRI composition, and wherein the calorie content of the meal is about 500 kcal or below.

In a fourth aspect, a meal for use in a method of reducing or preventing nausea, diarrhea, and/or headaches in a subject receiving an MRI composition is provided, the method comprising, separately or together, a physiologically acceptable manganese (II) compound, a proteinogenic amino acid and/or a vitamin D, and one or more water-soluble excipients; the method comprising:

i) administering a meal to the subject; and

ii) administering the MRI composition to the subject;

wherein the meal is administered from 0 to 8 hours prior to or up to about 2 hours subsequent to administering the MRI composition, and wherein the calorie content of the meal is about 500 kcal or below.

### *Definitions*

The term "administration" as used herein refers to the act of the MRI composition entering the body of a subject. Hence, time calculations prior to, or subsequent to administration are calculated in relation to the point in time where the MRI composition enters the body of a subject.

The term "meal", "predefined meal", "snack" or "light meal" as used herein interchangeably refer to a food serving having a calorie content of about a predefined value or below, such as about 500 kcal or below. The predefined value refers to a calorie content which is significantly below the calorie content of a full meal having a calorie content of about 900 kcal or higher.

The term "water-soluble" is used herein to describe a compound that dissolve in water at room temperature, such as about 25 °C. The extent of water-solubility ranges widely, from infinitely soluble (without limit) (fully miscible) such as ethanol in water, to poorly soluble, such as silver chloride in water. The term insoluble is often applied to poorly or very poorly soluble compounds.

The term "water-insoluble" is used herein to describe a compound that does not dissolve in water at room temperature, such as about 25 °C.

A number of other descriptive terms are also used to quantify the extent of solubility for a given solute, i.e. the compound subject to solution:

| Term | Mass parts of water required to dissolve 1 mass part of solute |
|---|---|
| Very soluble | <1 |
| Freely soluble | 1 to 10 |
| Soluble | >10 to 30 |
| Sparingly soluble | >30 to 100 |
| Slightly soluble | >100 to 1000 |
| Very slightly soluble | >1000 to 10,000 |
| Practically insoluble or insoluble | ≥ 10,000 |

The water-solubility of a given solute typically depends on temperature. Depending on the nature of the solute the solubility may increase or decrease with temperature. For most solids and liquids, their solubility in water increases with temperature.

The term "hygroscopic" is used herein to describe a compound that sorbs water, either by absorption, adsorption, or a combination of the two processes at between 40 and 60% relative humidity (rH) to any significant extent, such as to the extent wherein the mass and/or volume of said compound increases by about 5% or more within a period of time, such as after about 1 hour.

The term "non-hygroscopic water soluble" is used herein to describe a compound that is not hygroscopic in accordance with the definition herein, and at the same time is water-soluble according to the definition herein.

The term "absorption promoter" is used herein interchangeably with the term "uptake promoter" which refers to a compound capable of enhancing manganese transport across the membranes of the gastrointestinal tract. These compounds are well known in the art from e.g. WO 96/05867 and comprise physiologically tolerable reducing compounds containing an $\alpha$-hydroxy ketone group, a physiologically tolerable acid containing $\alpha$- and/or $\beta$-hydroxy or amino groups, or a salt thereof, and/or vitamin D.

### Description of Drawings

**Fig. 1:** Overview of study design from Example 1 with two periods of fasting and two groups of subjects receiving a full meal or a snack prior to dosing with Orviglance.

**Fig. 2:** Relative change in MRI signal intensity (SI%) from liver imaging at 1, 4, 8 and 24 hours post-dose for fasting subjects (black lines) compared to the same subjects after receiving a full meal (dotted lines). The SI% obtained from

imaging of the subjects after a full meal is significantly lower than after the fasting period. The contents of the full meal is further described in Table 1.

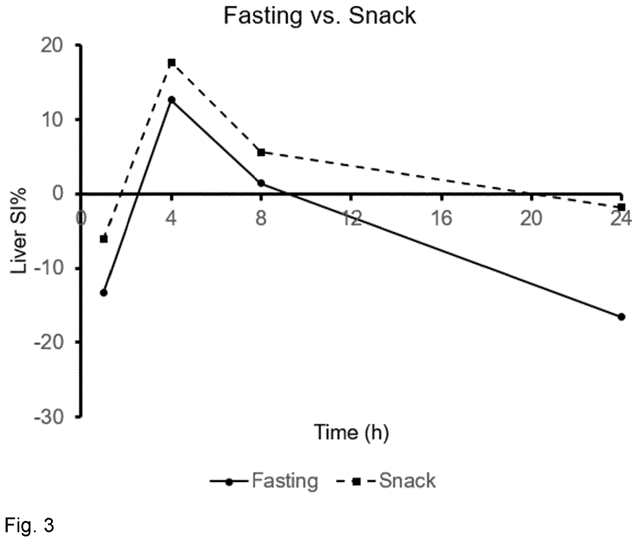

**Fig. 3:** Relative change in MRI signal intensity (SI%) from liver imaging at 1, 4, 8 and 24 hours post-dose for fasting subjects (black lines) compared to the same subjects after receiving a snack (dotted lines). The SI% obtained from imaging of the subject after the snack is surprisingly at least as good as after fasting. The contents of the snack is further described in Table 2.

## Detailed description

The invention is defined by the claims. Any subject matter falling outside the scope of the claims is provided for information purposes only. The references to methods of treatment in this description are to be interpreted as references to the compounds, pharmaceutical compositions and medicaments of the present invention for use in a method for the treatment of the human (or animal) body by therapy (or for diagnosis)

In one embodiment, an MRI composition is provided comprising, separately or together, a physiologically acceptable manganese (II) compound, a proteinogenic amino acid and/or a vitamin D, and optionally one or more water-soluble excipients; wherein the MRI composition is for use in the diagnosis in vivo of liver metastases and/or primary liver cancer in a subject; wherein the subject has consumed a meal within 8 hours prior to or up to about 2 hours subsequent to administration of the MRI composition and wherein the calorie content of the meal is about 500 kcal or below.

In one embodiment, use of the MRI composition reduces or prevents nausea, diarrhea, and/or headaches in the subject as compared to a fasting subject that has not consumed food for 3 hours or more prior to administration of the MRI composition. That is to say in one embodiment the subject has reduced nausea, and/or diarrhea, and/or headaches as compared to a fasting subject having been administered said MRI composition.

In one embodiment, the subject has not been fasting for 3 or more hours prior to the administration of the MRI composition.

### *Subjects negatively affected by fasting*

In addition to fasting being unpleasant or inconvenient, some subjects may be negatively affected by fasting for health reasons. For these subjects, the MRI composition and its use described herein are particularly well suited. Hence, in one embodiment, MRI composition is provided for use with a subject which is negatively affected by not eating for more than 8 hours.

In one embodiment, the subject suffers from a disease where not eating for more than 8 hours imposes a health risk to the subject. Hence, in some embodiments, the present disclosure provides an MRI composition for use with a subject which has not been eating for more than 8 hours prior to administration of the MRI composition.

In one embodiment, the subject has one or more of:

a. diabetes, such as diabetes type-I or diabetes type-II;
b. a Body Mass Index (BMI) of 20 or below;
c. an age of 60 years or above; and/or
d. hyperthyroidism.

### *Timing*

In one embodiment, the subject has consumed the meal from about 0 to 8 hours prior to or up to about 2 hours subsequent to administration of the MRI composition, such as from about 0 to 1 hour, such as from about 1 to 2 hours, such as from about 2 to 3 hours, such as from about 3 to 4 hours, such as from about 4 to 5 hours, such as from about 5 to 6 hours, such as from about 6 to 7 hours, such as from about 7 to 8 hours prior to administration of the MRI composition. In one embodiment, the subject has consumed the meal from up to about 30 minutes subsequent to, such as up to about 1 hour subsequent to, such as up to about 2 hours subsequent to administration of the MRI composition.

In one embodiment, the subject has consumed the meal within about 1 hour prior to administration of the MRI composition, such as within about 45 minutes, such as within about 30 minutes prior to administration of the MRI composition.

In one embodiment, the subject undergoes MRI from about 30 minutes to about 8 hours after administration of the MRI composition, such as from 2 hours to 3 hours, such as from 3 hours to 4 hours, such as from 4 hours to 5 hours, such as from 5 hours to 6 hours, such as from 6 hours to 7 hours, such as from 7 hours to 8 hours after administration of the MRI composition. In one particular embodiment, the subject undergoes MRI about 4 hours after administration of the MRI composition.

In one embodiment, the MRI composition is provided comprising, separately or together, a physiologically acceptable manganese (II) compound, a proteinogenic amino acid and/or a vitamin D, and optionally one or more water-soluble excipients; wherein the MRI composition is for use in the diagnosis in vivo of liver metastases and/or primary liver cancer in a subject; wherein the subject has consumed a meal within 2 hours prior to administration of the MRI composition and wherein the calorie content of the meal is about 500 kcal or below, and wherein the subject undergoes MRI about from 30 minutes to 6 hours, such as 4 hours after administration of the MRI composition.

***Meal having a calorie content about 500 kcal or below***

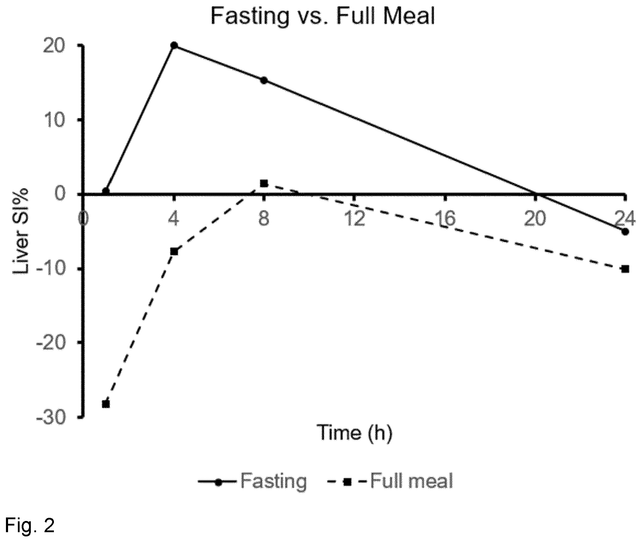

Some meals contain too many calories, such as more than 500 kcal, such as more than 600 kcal, such as more than 700 kcal, such as more than 800 kcal, such as more than 900 kcal and significantly interfere with the signal intensity enhancement during MRI as is demonstrated in Example 1 and as shown on Fig. 2. Such negative food effect on imaging was also recognized in the art, by Leander et al (Investigative Radiology, 45, 2010). An example of a meal, referred to herein as a full mean, which significantly negatively impact the MRI quality is shown in Table 1.

**Table. 1:** Contents of a full meal.

| ***Food Items*** | ***Servings*** | ***Ingredients*** | ***Qty. Of Ingredients*** | **Calorie** (Kcal) | **CHO** (gms) | **Fats** (gms) | **Proteins** (gms) |
|---|---|---|---|---|---|---|---|
| Toast | 1 no | Bread | 1 Slice | 61.25 | 12.98 | 0.18 | 1.95 |
| | | Butter | 10 gms | 72.90 | 0.00 | 8.10 | 0.00 |
| *Chana Chat | 60 gms | Chana | 20 gms | 72.00 | 12.18 | 1.06 | 3.42 |
| | | Onions | 5 gms | 2.95 | 0.63 | 0.01 | 0.09 |
| | | Peanuts | 10 gms | 56.7 | 2.61 | 4.01 | 2.53 |
| | | Oil | 2 ml | 18.00 | 0.00 | 2.00 | 0.00 |
| Vegetable Cutlets (Approx 130 gms) | 1 serving / 2 nos | Potatoes | 50 gms | 48.50 | 11.30 | 0.05 | 0.80 |
| | | Cheese | 40 gms | 139.20 | 2.52 | 10.04 | 9.64 |
| | | Onions | 5 gms | 2.95 | 0.63 | 0.01 | 0.09 |
| | | Bread | ¼ Slice | 15.31 | 3.24 | 0.04 | 0.49 |
| | | Oil | 10 ml | 90.00 | 0.00 | 10.00 | 0.00 |
| | | Paneer | 50 gms | 146.00 | 3.95 | 11.50 | 6.70 |
| Milk# | 1 serving | Milk | 200 ml | 192.00 | 10.00 | 13.00 | 8.60 |
| | | Sugar | 5 gms | 19.90 | 4.97 | 0.00 | 0.01 |
| **TOTAL** | | | | **937.66** | **65.01** | **60.00** | **34.32** |
| NUTRIENT CALORIES | | | | | **260.04** | **540.00** | **137.28** |
| **NUTRIENT CALORIES AS % TOTAL** | | | | | **27.73%** | **57.59%** | **14.64%** |

In one embodiment, the meal according to the present disclosure having a calorie content about 500 kcal or below is characterized as a snack or a light meal. An exemplary overview of the contents of the meal having a calorie content about 500 kcal or below is provided in Table 2.

**Table: 2:** Contents of a snack.

| ***Food Items*** | ***Servings*** | ***Ingredients*** | ***Oty. Of Ingredients*** | ***Calorie (Kcal)*** | ***CHO (gm)*** | ***Fats (gm)*** | ***Proteins (gm)*** |
|---|---|---|---|---|---|---|---|
| **Toast** | 1 slice | Bread | 1 slice | 61.25 | 12.98 | 0.18 | 1.95 |
| | | Butter | 10 gms | 72.90 | 0.00 | 8.10 | 0.00 |
| **Sweet Lime Juice** | 200 ml | Sweet Lime Pulp | 50 gm | 13.64 | 2.59 | 0.1 | 0.38 |
| | | sugar | 15 gm | 59.7 | 14.91 | 0.00 | 0.02 |

(continued)

| Food Items | Servings | Ingredients | Oty. Of Ingredients | Calorie (Kcal) | CHO (gm) | Fats (gm) | Proteins (gm) |
|---|---|---|---|---|---|---|---|
| Total | | | | 207.49 | 30.48 | 8.38 | 2.35 |
| Nutrients Calories | | | | | 121.90 | 75.38 | 9.40 |
| Nutrients Calories as % of total | | | | | 58.7% | 36.3% | 4.5% |

In the context of the present disclosure, "the meal" refers to the total calorie intake during the period specified in the present disclosure, such as the total calorie intake from about 0 to 8 hours prior to or up to about 2 hours subsequent to administration of the MRI composition.

In some embodiments, the meal constitutes a plurality of food items, such as a toast with butter and a juice. In some embodiments, the subject has consumed no more than about 500 kcal from about 0 to 8 hours prior to or up to about 2 hours subsequent to administration of the MRI composition. In some embodiments, the subject has consumed no more than about 400 kcal from about 0 to 8 hours prior to or up to about 2 hours subsequent to administration of the MRI composition. In some embodiments, the subject has consumed no more than about 300 kcal from about 0 to 8 hours prior to or up to about 2 hours subsequent to administration of the MRI composition.

In some embodiments, the subject has consumed no more than about 500 kcal from the time specified herein prior to or up to the time specified herein subsequent to administration of the MRI composition.

In one embodiment, the meal comprises a predefined amount of carbohydrates, a predefined amount of fats, and a predefined amount of proteins.

In one embodiment, the predefined amount of carbohydrates in the meal is from 20 gram to 50 gram, such as from 20 to 22 gram, such as from 22 to 24 gram, such as from 24 to 26 gram, such as from 26 to 28 gram, such as from 28 to 30 gram, such as from 30 to 32 gram, such as from 32 to 34 gram, such as from 34 to 36 gram, such as from 36 to 38 gram, such as from 38 to 40 gram, such as from 40 to 42 gram, such as from 42 to 44 gram, such as from 44 to 46 gram, such as from 46 to 48 gram, such as from 48 to 50 gram.

In one embodiment, the predefined amount of carbohydrates in the meal is about 30 gram or about 31 gram.

In one embodiment, the predefined amount of carbohydrates in the meal is from 60 to 200 kcal, such as from 60 to 70 kcal, such as from 70 to 80 kcal, such as from 80 to 90 kcal, such as from 90 to 100 kcal, such as from 100 to 110 kcal, such as from 110 to 120 kcal, such as from 120 to 130 kcal, such as from 130 to 140 kcal, such as from 140 to 150 kcal, such as from 150 to 160 kcal, such as from 160 to 170 kcal, such as from 170 to 180 kcal, such as from 180 to 190 kcal, such as from 190 to 200 kcal.

In one embodiment, the predefined amount of carbohydrates in the meal is from 50 to 70 kcal% of the total amount of calories in the meal, such as from 50 to 52 kcal%, such as from 52 to 54 kcal%, such as from 54 to 56 kcal%, such as from 56 to 58 kcal%, such as from 58 to 60 kcal%, such as from 60 to 62 kcal%, such as from 62 to 64 kcal%, such as from 64 to 66 kcal%, such as from 66 to 68 kcal%, such as from 68 to 70 kcal% of the total amount of calories in the meal.

In one embodiment, the predefined amount of carbohydrates in the meal is about 59% of the total amount of calories in the meal.

In one embodiment, the predefined amount of fats in the meal is from 5 gram to 30 gram, such as from 5 to 10 gram, such as from 10 to 15 gram, such as from 15 to 20 gram, such as from 20 to 25 gram, such as from 25 to 30 gram.

In one embodiment, the predefined amount of fats in the meal is about 7 gram, about 8 gram, about 9 gram, or about 10 gram.

In one embodiment, the predefined amount of fats in the meal is from 30 to 150 kcal, such as from 30 to 40 kcal, such as from 40 to 50 kcal, such as from 50 to 60 kcal, such as from 60 to 70 kcal, such as from 70 to 80 kcal, such as from 80 to 90 kcal, such as from 90 to 100 kcal, such as from 100 to 110 kcal, such as from 110 to 120 kcal, such as from 120 to 130 kcal, such as from 130 to 140 kcal, such as from 140 to 150 kcal.

In one embodiment, the predefined amount of fats in the meal is from 25 to 45 kcal% of the total amount of calories in the meal, such as from 25 to 27 kcal%, such as from 27 to 29 kcal%, such as from 29 to 31 kcal%, such as from 31 to 33 kcal%, such as from 33 to 35 kcal%, such as from 35 to 37 kcal%, such as from 37 to 39 kcal%, such as from 39 to 41 kcal%, such as from 41 to 43 kcal%, such as from 43 to 45 kcal% of the total amount of calories in the meal.

In one embodiment, the predefined amount of fats in the meal is about 36% of the total amount of calories in the meal.

In one embodiment, the predefined amount of proteins in the meal is from 0 gram to 10 gram, such as from 0 to 2 gram, such as from 2 to 4 gram, such as from 4 to 6 gram, such as from 6 to 8 gram, such as from 8 to 10 gram.

In one embodiment, the predefined amount of proteins in the meal is about 0 gram, about 1 gram, about 2 gram, or about 3 gram.

In one embodiment, the predefined amount of proteins in the meal is from 0 to 50 kcal, such as from 0 to 5 kcal,

such as from 5 to 10 kcal, such as from 10 to 15 kcal, such as from 15 to 20 kcal, such as from 20 to 25 kcal, such as from 25 to 30 kcal, such as from 30 to 35 kcal, such as from 35 to 40 kcal, such as from 40 to 45 kcal, such as from 45 to 50 kcal.

In one embodiment, the predefined amount of proteins in the meal is from 0 to 10 kcal% of the total amount of calories in the meal, such as from 0 to 1 kcal%, such as from 1 to 2 kcal%, such as from 2 to 3 kcal%, such as from 3 to 4 kcal%, such as from 4 to 5 kcal%, such as from 5 to 6 kcal%, such as from 6 to 7 kcal%, such as from 7 to 8 kcal%, such as from 8 to 9 kcal%, such as from 9 to 10 kcal% of the total amount of calories in the meal.

In one embodiment, the predefined amount of proteins in the meal is about 5% of the total amount of calories in the meal.

In one embodiment, the meal comprises one or more food items selected from the group consisting of: bread, such as toast bread with butter, and fruit juice, such as sweet lime juice.

In one embodiment, the meal comprises one or more sugars selected from the list consisting of: glucitol, mannitol, glycerol, glycol, $\alpha$-glucose, $\alpha$-galactose, $\alpha$-xylose, $\beta$-glucose, $\beta$-galactose, $\beta$-xylose, $\beta$-arabinose and sucrose.

In one embodiment, the juice is provided at a volume of from 100 mL to 400 mL, such as from 100 mL to 110 mL, such as from 110 mL to 120 mL, such as from 120 mL to 130 mL, such as from 130 mL to 140 mL, such as from 140 mL to 150 mL, such as from 150 mL to 160 mL, such as from 160 mL to 170 mL, such as from 170 mL to 180 mL, such as from 180 mL to 190 mL, such as from 190 mL to 200 mL, such as from 200 mL to 210 mL, such as from 210 mL to 220 mL, such as from 220 mL to 230 mL, such as from 230 mL to 240 mL, such as from 240 mL to 250 mL, such as from 250 mL to 260 mL, such as from 260 mL to 270 mL, such as from 270 mL to 280 mL, such as from 280 mL to 290 mL, such as from 290 mL to 300 mL, such as from 300 mL to 310 mL, such as from 310 mL to 320 mL, such as from 320 mL to 330 mL, such as from 330 mL to 340 mL, such as from 340 mL to 350 mL, such as from 350 mL to 360 mL, such as from 360 mL to 370 mL, such as from 370 mL to 380 mL, such as from 380 mL to 390 mL, such as from 390 mL to 400 mL.

In one embodiment, the calorie content of the meal is from 100 kcal to 500 kcal, such as from 100 to 110 kcal, such as from 110 kcal to 120 kcal, such as from 120 kcal to 130 kcal, such as from 130 kcal to 140 kcal, such as from 140 kcal to 150 kcal, such as from 150 kcal to 160 kcal, such as from 160 kcal to 170 kcal, such as from 170 kcal to 180 kcal, such as from 180 kcal to 190 kcal, such as from 190 kcal to 200 kcal, such as from 200 kcal to 210 kcal, such as from 210 kcal to 220 kcal, such as from 220 kcal to 230 kcal, such as from 230 kcal to 240 kcal, such as from 240 kcal to 250 kcal, such as from 250 kcal to 260 kcal, such as from 260 kcal to 270 kcal, such as 270 kcal to 280 kcal, such as from 280 kcal to 290 kcal, such as from 290 kcal to 300 kcal, such as from 300 kcal to 310 kcal, such as from 310 kcal to 320 kcal, such as from 320 kcal to 330 kcal, such as from 330 kcal to 340 kcal, such as from 340 kcal to 350 kcal, such as from 350 kcal to 360 kcal, such as from 360 kcal to 370 kcal, such as from 370 kcal to 380 kcal, such as from 380 kcal to 390 kcal, such as from 390 kcal to 400 kcal, such as from 400 kcal to 410 kcal, such as from 410 kcal to 420 kcal, such as from 420 kcal to 430 kcal, such as from 430 kcal to 440 kcal, such as from 440 kcal to 450 kcal, such as from 450 kcal to 460 kcal, such as from 460 kcal to 470 kcal, such as from 470 kcal to 480 kcal, such as from 480 kcal to 490 kcal, such as from 490 kcal to 500 kcal.

In a particular embodiment, the meal has a calorie content of about 300 kcal or below, and the predefined amount of carbohydrates in the meal constitutes at least 50% of the calorie content of the meal.

In some embodiments, the meal employed has a calorie content of about 300 kcal or below, and the predefined amount of carbohydrates in the meal constitutes at least 50% of the calorie content of the meal, and the subject has consumed the meal from 0 to 4 hours prior to administration of the MRI composition, such as from 0 to 1 hours, such as from 1 to 2 hours, such as from 2 to 3 hours, such as from 3 to 4 hours prior to the administration of the MRI composition.

In some embodiments, the meal does not comprise certain food items, metal ions, or metals as specified herein below.

In some embodiments, the meal does not comprise xanthine or derivatives thereof, such as caffeine. In some embodiments, the meal does not comprise one or more food items selected from the group consisting of: chocolate, tea, coffee, cola, and tobacco. In some embodiments, the meal does not comprise dietary supplementation, such as vitamin supplementation.

In some embodiments, the meal does not comprise metal ions, such as metal irons derived from one or more of: calcium, chromium, copper, iron, magnesium, manganese, molybdenum, potassium, sodium and zinc.

In some embodiments, the meal does not comprise iron in an amount of 10 mg or more, such as 15 mg or more.

In some embodiments, the meal does not comprise magnesium in an amount of 100 mg or more, such as 200 mg or more, such as 300 mg or more, such as 400 mg or more.

In some embodiments, the meal does not comprise calcium in an amount of 500 mcg or more, such as 1000 mcg or more.

In some embodiments, the meal does not comprise calcium, magnesium and/or iron in the amounts specified herein.

In some embodiments, the meal does not comprise one or more of magnesium-containing antacids and one or more laxatives. In some embodiments, the meal does not comprise grapefruit or food products derived from grapefruit. In some embodiments, the meal does not comprise ethanol.

In some embodiments, the meal does not comprise one or more of the food items, metal ions, or metals in any

combination or amounts specified herein.

### *Methods and effects*

In one embodiment, a method for MRI of a subject is provided comprising, administering a composition comprising separately or together, a physiologically acceptable manganese (II) compound, a proteinogenic amino acid and/or a vitamin D, and optionally one or more water-soluble excipients to the subject; and performing MRI on said subject; wherein the subject has consumed a meal from 0 to 8 hours prior to or up to 2 hours subsequent to administration of the MRI composition, and wherein the calorie content of the meal is about 500 kcal or below.

The MRI is usually performed after a defined period of time following administration of the MRI composition to the subject as described elsewhere herein.

In one embodiment, a meal for use in a method of reducing or preventing nausea, diarrhea, and/or headaches in a subject receiving an MRI composition is provided, the method comprising, separately or together, a physiologically acceptable manganese (II) compound, a proteinogenic amino acid and/or a vitamin D, and optionally one or more water-soluble excipients; the method comprising:

i) administering a meal to the subject; and
ii) administering the MRI composition to the subject;

wherein the meal is administered from 0 to 8 hours prior to or up to 2 hours subsequent to administering the MRI composition, and wherein the calorie content of the meal is about 500 kcal or below.

In one embodiment, the meal is administered from 0 to 7 hours prior to the administering of the MRI composition, such as from 0 to 1 hour, such as from 1 to 2 hours, such as from 2 to 3 hours, such as from 3 to 4 hours, such as from 4 to 5 hours, such as from 5 to 6 hours, such as from 6 to 7 hours prior to the administering of the MRI composition.

In one embodiment, the imaging quality is unaffected compared to a method where the MRI composition is administered to a fasting subject, wherein the fasting subject has not been eating for more than 8 hours prior to administration of the MRI composition nor before MRI. Imaging quality is in some embodiments assessed in terms of signal intensity SI(%) enhancement.

### *MRI composition*

In one embodiment, the MRI composition comprises, separately or together, a physiologically acceptable manganese (II) compound, a proteinogenic amino acid and/or a vitamin D, and optionally one or more water-soluble excipients.

### *Physiologically acceptable manganese (II) compounds*

In one embodiment, the MRI composition is provided as defined herein, wherein the physiologically acceptable manganese (II) compound is a salt of an inorganic anion or an organic anion. In one embodiment, the inorganic anion is selected from the group consisting of chloride, fluoride, bromide, iodide, sulphate, and phosphate. In one embodiment, the organic anion is selected from the group consisting of: ascorbate, kojate, salicylate, and gluconate.

In one embodiment, the MRI composition is provided as defined herein, wherein the physiologically acceptable manganese (II) compound is selected from the group consisting of: manganese (II) sulphate, manganese (II) gluconate, manganese (II) chloride anhydrate, manganese (II) chloride dihydrate, manganese (II) chloride tetrahydrate, and a combination thereof.

In one embodiment, the MRI composition is provided as defined herein, wherein the physiologically acceptable manganese (II) compound is manganese (II) chloride tetrahydrate or another hydrate of manganese (II) chloride.

In one embodiment, the MRI composition is provided as defined herein, wherein the MRI composition comprises between 0.5 g and 1.2 g manganese (II) chloride tetrahydrate or an equimolar amount of any corresponding manganese (II) salt.

In one embodiment, the MRI composition is provided as defined herein, wherein the MRI composition comprises between 0.6 g and 1 g of manganese (II) chloride tetrahydrate, such as between 0.65 g and 0.95 g, such as between 0.70 g and 0.90 g, such as between 0.75 g and 0.85 g, such as 0.80 g.

In one embodiment, the MRI composition is provided as defined herein, wherein the MRI composition comprises 0.8 g manganese (II) chloride tetrahydrate or an equimolar amount of any corresponding manganese (II) salt.

In one embodiment, the MRI composition is provided as defined herein, wherein the MRI composition comprises 0.8 g manganese (II) chloride tetrahydrate or an equimolar amount of the corresponding anhydrate or dihydrate.

In some embodiments, the MRI composition is Orviglance comprising: 800 mg manganese (II) chloride

tetrahydrate, 500 mg L-alanine, and 800 IU vitamin D3. Orviglance has previously been known as Mangoral. In some embodiments, the MRI composition further comprises one or more water-soluble excipients.

In some embodiments, the MRI composition comprises: manganese (II) chloride tetrahydrate, L-alanine, and vitamin D3. In some embodiments, the MRI composition further comprises one or more water-soluble excipients.

In some embodiments, the MRI composition comprises: manganese (II) chloride or any hydrate thereof, L-alanine, and vitamin D3. In some embodiments, the MRI composition further comprises one or more water-soluble excipients.

***Pharmaceutical excipients***

A pharmaceutical excipient is a substance formulated alongside the active ingredient of a medicament, included for the purpose of long-term stabilization, bulking up solid formulations that contain potent active ingredients in small amounts (thus often referred to as "bulking agents", "fillers", or "diluents"), or to confer a therapeutic enhancement on the active ingredient in the final dosage form, such as but not limited to facilitating drug absorption, reducing viscosity, or enhancing solubility. Pharmaceutical excipients can also be useful in the manufacturing process, to aid in the handling of the active substance concerned such as by facilitating powder flowability or non-stick properties, in addition to aiding in vitro stability such as prevention of denaturation or aggregation over the expected shelf life.

It will be understood by a person of skill in the art, that while some excipients are preferred and some are not preferred to include in the compositions as disclosed herein, it may be possible to produce an MRI composition as defined herein (ie provided together or separate) non-preferred excipient if the relative amount of that excipient is carefully controlled.

In one embodiment, the MRI composition is provided as defined herein, wherein the one or more water-soluble excipients are non-hygroscopic water-soluble excipients. In one embodiment, the one or more water-soluble excipients are selected from the group consisting of: a non-hygroscopic filler, a non-hygroscopic binder, a non-hygroscopic disintegrant, and a non-hygroscopic lubricant.

Fillers/diluents: a "filler" or a "diluent" according to the present disclosure is preferably a water-soluble and non-hygroscopic filler, but may in some embodiments also be a hygroscopic filler. In one embodiment, the MRI composition is provided as defined herein, wherein the non-hygroscopic filler is selected from the group consisting of: isomalt; lactose, such as spray-dried lactose, a-lactose, or β-lactose; maltitol; maltose; and mannitol.

Dextrin and lactitol may be used as alternative fillers in low amounts but typically contain 5% moisture.

Dextrates, dextrose, fructose, hypromellose, maltodextrin, sucrose, sorbitol and xylitol are also water-soluble fillers but are all hygroscopic. These may thus be used in some embodiments where hygroscobicity is not a relevant issue, such as when the MRI composition is formulated in separate compartments. In some embodiments, the MRI composition is provided as defined herein, wherein the MRI composition comprise a hygroscopic filler selected from the group consisting of: a dextrate, dextrose, fructose, hypromellose, maltodextrin, sucrose, sorbitol and xylitol.

In some embodiments, the MRI composition may comprise a water-insoluble filler such as selected from the group consisting of: calcium carbonate, calcium phosphate (e.g. basic calcium phosphate, calcium hydrogen phosphate, dicalcium phosphate), calcium silicate, cellulose powdered, cellulose acetate, magnesium carbonate, magnesium oxide, medium-chain triglycerides, microcrystalline cellulose, silicified microcrystalline cellulose, polymethacrylates, starch, calcium sulfate, and pregelatinized starch. In other embodiments, the MRI composition is provided as defined herein, wherein the MRI composition does not comprise a water-insoluble filler selected from the group consisting of calcium carbonate; calcium phosphate, such as basic calcium phosphate, calcium hydrogen phosphate, or dicalcium phosphate; calcium silicate; cellulose powder; cellulose acetate; magnesium carbonate; magnesium oxide; a medium-chain triglyceride; microcrystalline cellulose; silicified microcrystalline cellulose; a polymethacrylate; starch; calcium sulfate; and pregelatinized starch.

Binders: a "binder" according to the present disclosure is preferably a water-soluble and non-hygroscopic binder. In one embodiment, the water-soluble and non-hygroscopic binder is selected from the group consisting of: hydroxyethylmethyl cellulose, maltose, povidone, and dextrin. In one embodiment, the binder is povidone. Binders are in particular of relevance when the contents of the MRI composition as defined herein in some embodiments are formulated together such as in a tablet.

Carboxymethylcellulose sodium, dextrates, dextrose, hydroxyethyl cellulose, hydroxypropyl cellulose, maltodextrin, poloxamer, polydextrose and sucrose may be used as alternative water-soluble binders in low amounts but are all hygroscopic.

In one embodiment, the MRI composition is provided as defined herein, wherein the MRI composition does not comprise a poorly water-soluble binder selected from the group consisting of: acacia, hypromellose, methylcellulose, sodium alginate, pregelatinized starch, inulin, agar, gelatin, starch, alginic acid, cellulose acetate phthalate, ethylcellulose, hydrogenated vegetable oil, magnesium aluminum silicate, microcrystalline cellulose, and polymethacrylate.

Disintegrants: a "disintegrant" is known in the art to expand and dissolve when wet causing a compressed

pharmaceutical composition, such as a tablet, to break apart.

Disintegrants are thus in particular of relevance when the MRI composition as defined herein is formulated together such as in a tablet.

Povidone is a water-soluble disintegrant, but not a very strong disintegrant. In some embodiments, the MRI composition is provided comprising a non-hygroscopic disintegrant, wherein the non-hygroscopic disintegrant is povidone.

Carboxymethylcellulose calcium, croscarmellose sodium, and crospovidone are insoluble in water, but very strong disintegrants and may be applied in very low levels and are preferred. In some embodiments, the MRI composition is provided as defined herein, further comprising a water-insoluble disintegrant, such as selected from the group consisting of: carboxymethylcellulose calcium; croscarmellose sodium; and crospovidone.

Carboxymethylcellulose sodium and hydroxypropyl cellulose are examples of hygroscopic water-soluble disintegrants. These may thus be used in some embodiments where hygroscobicity is not a relevant issue, such as when the MRI composition is formulated in separate compartments. In one embodiment, the MRI composition comprise a hygroscopic disintegrant selected from the group consisting of: carboxymethylcellulose sodium and hydroxypropyl cellulose.

Lubricants: A "lubricant" prevents ingredients from sticking to and clogging production machinery, such as filling tubes and tablet punches. E.g. during tablet production, lubricants also ensure that tablet formation and ejection can occur with low friction between the solid and die wall.

Polyethylene glycol 6000 and sodium benzoate are water-soluble and non-hygroscopic binders. In one embodiment, the MRI composition is provided as defined herein, wherein the non-hygroscopic lubricant is selected from the group consisting of: polyethylene glycol 6000 and sodium benzoate.

Sodium lauryl sulfate yields a bitter taste and hence, is not preferred.

Poloxamer and polyethylene glycol 4000 are water-soluble but hygroscopic lubricants and may thus be used in some embodiments where hygroscobicity is not a relevant issue, such as when the MRI composition is formulated in separate compartments. In some embodiments, the MRI composition is provided as defined herein, wherein the MRI composition comprise a hygroscopic lubricant selected from the group consisting of: poloxamer and polyethylene glycol 4000.

Sodium stearyl fumarate, calcium stearate, colloidal silica, glycerin monostearate, glyceryl behenate, glyceryl palmitostearate, hydrogenated castor oil, hydrogenated vegetable oil, magnesium stearate, medium-chain triglycerides, palmitic acid, stearic acid, talc, zinc stearate are all water-insoluble and thus not preferred to include in the MRI composition as defined herein. In one embodiment, the MRI composition does not comprise a water-insoluble lubricant selected from the group consisting of: sodium stearyl fumarate, calcium stearate, colloidal silica, glycerin monostearate, glyceryl behenate, glyceryl palmitostearate, hydrogenated castor oil, hydrogenated vegetable oil, magnesium stearate, medium-chain triglycerides, palmitic acid, stearic acid, talc, and zinc stearate.

***Absorption promoters***

Under physiological circumstances the manganese of the physiologically acceptable manganese (II) compound is poorly absorbed from the intestine after oral intake, but by the use of specific absorption promoters, such as L-alanine and vitamin $D_3$, it is possible to obtain a sufficiently high concentration in the liver in order to achieve a significant signal enhancing effect during MRI.

The term "absorption promoter" is used herein interchangeably with the term "uptake promoter" which refers to a compound capable of enhancing manganese transport across the membranes of the gastrointestinal tract. These compounds are well known in the art from e.g. WO 96/05867 and comprise physiologically tolerable reducing compounds containing an $\alpha$-hydroxy ketone group, a physiologically tolerable acid containing $\alpha$- and/or $\beta$-hydroxy or amino groups, or a salt thereof, and/or vitamin D.

In one embodiment, the MRI composition is provided as defined herein, wherein the one or more absorption promotors are selected from the group consisting of: a proteinogenic amino acid and a vitamin D.

In one embodiment, the MRI composition is provided as defined herein, wherein the proteinogenic amino acid is selected from the group consisting of alanine, valine, leucine, tryptophan, methionine, isoleucine, proline, phenylalanine, serine, glycine, threonine, cysteine, asparagine, glutamine, tyrosine, aspartic acid, glutamic acid, arginine, lysine and histidine.

In one embodiment, the MRI composition is provided as defined herein, wherein the proteinogenic amino acid is a hydrophobic amino acid selected from the group consisting of alanine, glcine, valine, leucine, isoleucine, proline, phenylalanine, methionine and tryptophan.

In one embodiment, the MRI composition is provided as defined herein, wherein the proteinogenic amino acid is a neutral amino acid.

In one embodiment, the MRI composition is provided as defined herein, wherein the proteinogenic amino acid is

L-alanine.

In one embodiment, the MRI composition is provided as defined herein, wherein the vitamin D is vitamin $D_3$.

In one embodiment, the MRI composition is provided as defined herein, comprising two absorptions promoters. In one embodiment, the two absorption promoters are L-alanine and vitamin $D_3$.

In one embodiment, the MRI composition is provided as defined herein, wherein the MRI composition comprises between 0.25 g and 0.75 g of a proteinogenic amino acid, such as L-alanine, such as between 0.30 g and 0.70 g, such as between 0.35 g and 0.65 g, such as between 0.40 g and 0.60 g, such as between 0.45 g and 0.55 g, such as 0.50 g.

## Examples

### Example 1: Study of Orviglance food effects

Materials

Active Treatment

IMP: Manganese (II) chloride tetrahydrate (MnCl2 ▪ 4H2O; working name: Orviglance) powder for oral administration. Single oral dose of Orviglance (800 mg manganese (II) chloride tetrahydrate, 500 mg L-alanine, and 800 IU vitamin D3).

General Pharmacology:

Orviglance is an oral liver imaging drug (i.e. a liver specific contrast agent) being developed for detection and visualization of focal liver lesions, using Magnetic Resonance Imaging ("MRI") in patients where use of gadolinium-based contrast agents ("GBCAs") may be medically inadvisable or cannot be administered.

Overall objective:

To evaluate effects of food on Orviglance after a single oral administration in adult healthy subjects. To assess the PK and PD of Orviglance in healthy human subjects in fasting and fed (full meal or snacks) conditions.

Method

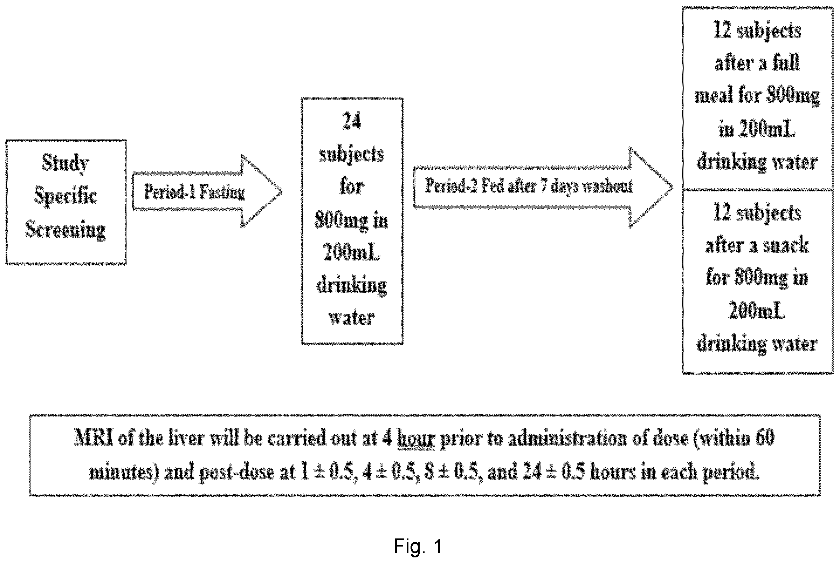

Period-1 was under fasting condition and Period-2 was under fed condition (full meal according to Table 1, or a snack according to Table 2). A flow chart of the study design is also depicted in Fig. 1. The rationale for the study design was to allow evaluation of orally administered Orviglance under fasting and fed conditions (full meal or snack), respectively by assessing Pharmacokinetics (PK) and Pharmacodynamics (PD) in healthy adult human subjects c.f. Fig 1.

Randomization

The allocation to receive light snack and full meal for each subject during second period of the study was determined according to a randomization schedule. The randomization schedule was generated using SAS® Version 9.4 or higher (SAS Institute Inc., USA) by the biostatistician. The sequence of administration of treatments i.e. "$T_1T_{21}$" or "$T_1T_{22}$" to the subjects was determined according to the randomization schedule as per below table 3. Equal allocation of subjects in each sequence was ensured.

**Table. 3:** overview of randomization schedule for Example 1.

| Sample size (N=24) | Period-1 (Fasting, N=24) | Period-2 (Fed, N=24) |
|---|---|---|
| Sequence $T_1T_{21}$ (N=12) | Test 800mg/200mL ($T_1$) | Test 800mg/200mL after full meal ($T_{21}$) |
| Sequence $T_1T_{22}$ (N=12) | | Test 800mg/200mL after snack ($T_{22}$) |

Number of Subjects

Maximum 32 subjects were enrolled in the study in order to obtain data of 24 completers who completed both fasting and fed (full meal and snack) study periods.

Housing

Subjects were housed in the clinical facility at least 60 hours before administration of the dose and continued to remain in the clinical facility for at least 72 hours after the administration of investigational medicinal product in all the periods.

Check-in procedures/Check-in activities were carried out as per in house procedure at each period. The subjects stayed in the facility for six consecutive nights in each period.

MRI of the liver was carried out at 4 hour prior to administration of dose (within 60 minutes) and post-dose at 1 $\pm$ 0.5, 4 $\pm$ 0.5, 8 $\pm$ 0.5, and 24 $\pm$ 0.5 hours utilizing imaging parameters.

Diet

All subjects were to abstain from xanthine containing food or beverages (like chocolates, tea, coffee or cola drinks), tobacco, products containing tobacco (like pan, pan masala, gutkha), for 24 hours prior to check-in till their stay in clinical facility. Dietary iron supplementation, including a multivitamin that contains iron, was prohibited from 6 hours prior to check-in of period-1 until after the last contrast-enhanced MRI of study. Supplemental magnesium and/or supplemental calcium may reduce manganese absorption and was prohibited from 6 hours prior to check-in of period-1 until after the last contrast-enhanced MRI of study.

Supplemental vitamin C or vitamin D3, which promote manganese absorption, were prohibited from 6 hours prior to check-in of period-1 until after the last contrast enhanced MRI of study. Magnesium-containing antacids and laxatives were prohibited from 6 hours prior to check-in of period-1 until after the last contrast-enhanced MRI of study. Quinolone and tetracycline antibiotics were prohibited from 6 hours prior to check-in of period-1 until after the last contrast-enhanced MRI of study.

All subjects were to abstain from grapefruit or its products within 72 hours prior to check-in of period-1 till last sample collection of study. All subjects were to abstain from alcohol or alcoholic products, recreational drugs within 48 hours prior to check-in of period-1 till last sample collection of study. Smoking for 6 months prior to check-in of period-1 till the completion of the study was prohibited and the subjects were to abstain from the same.

Postural Restriction

Orviglance was administered to the subjects while in sitting posture. Subjects were in sitting posture or ambulatory posture for the first 4 hours post-dose in each period. In case of adverse event(s), position of the subject was changed appropriately. Thereafter, the subjects were allowed to engage only in normal activities while avoiding any strenuous physical activity. Note: Subjects were in supine position at the time of MRI.

Duration of Fasting & Distribution of Meals:

**For period-1:**

An overnight fast of at least 4 hours prior to dosing in period-1 and lunch will be provided after completion of 4 hr post dose MRI.

**For period-2:**

All subjects will be required to fast overnight for at least 6 hours prior to serving of full meal according to Table 1 or a snack according to Table 2, which they are required to consume completely within 30 minutes of serving the same. Lunch will be provided after 4 $\pm$ 1 hours of dose administration in period-2 after. The dietician/trained study person weighed the leftover food and calculated the calories for any subjects that had not consumed the full meal or snacks completely. Standardized meals were served to the subjects at appropriate times during their stay in the clinical facility. The subjects received lunch at least 4 $\pm$ 1 hours (after 4 hours MRI) (for period-1 and for period-2) after dosing and further meals were served at appropriate intervals from then on, until checkout.

Subjects were to refrain from drinking water from 1 hour before till 1 hour after each dosing in each period except for 200 mL of water administered during dosing. Prior to and thereafter, water could be consumed as required.

**Results**

The results of the study are shown in below tables, Table 4 (comparing full meal vs fasting); and Table 5 (comparing the snack vs fasting). Optimal SI(%) enhancement is most often found about 4 hours post dosing; hence,

comparison of SI(%) at 4 hours post dosing is important to examine the difference in SI(%) between nonfasting and fasting subjects.

| Mean (SD) (T21 – full meal vs T1 - fasting) | | | | | | |
|---|---|---|---|---|---|---|
| **Statistics** | **SNR** | | **Normalised signal intensity** | | **Liver SI(%)enhancement** | |
| | Test (T1) | Test (T21) | Test (T1) | Test (T21) | Test (T1) | Test (T21) |
| **Baseline** | 37.9 (16.47) | 179.3 (403.05) | - | - | - | - |
| **at 1 hrs.** | 98.2 (217.87) | 129.1 (194.17) | 129.4 (490.21) | 148.4 (378.99) | 0.4 (24.31) | -28.2 (13.64) |
| **at 4 hrs.** | 208.0 (518.88) | 77.7 (88.12) | 381.0 (1172.65) | 27.0 (43.35) | **20.0 (16.34)** | **-7.7 (14.99)** |
| **at 8 hrs.** | 63.9 (56.75) | 119.5 (183.24) | 65.5 (129.17) | 20.4 (64.54) | 15.4 (25.20) | 1.4 (18.28) |
| **at 24 hrs.** | 118.2 (130.88) | 195.9 (321.88) | 239.8 (407.34) | 132.1 (260.89) | -5.0 (20.87) | -10.1 (18.23) |

**Table 4**. Relative change in MRI signal intensity (SI%) and signal intensity from liver imaging at 1, 4, 8 and 24 hours post-dose for fasting subjects (T1) compared to the same subjects after receiving a full meal (T21). The SI% obtained from imaging of the subjects after a full meal is significantly lower than after the fasting period.

| Mean (SD) (T22 - snack vs T1 - fasting) | | | | | | |
|---|---|---|---|---|---|---|
| **Statistics** | **SNR** | | **Normalised signal intensity** | | **Liver SI(%)enhancement** | |
| | Test (T1) | Test (T22) | Test (T1) | Test (T22) | Test (T1) | Test (T22) |
| **Baseline** | 53.7 (47.763) | 128.9 (208.00) | - | - | - | - |
| **at 1 hrs.** | 48.3 (32.044) | 91.8 (95.15) | 22.5 (86.28) | 5.3 (42.98) | -13.3 (20.92) | -6.1 (14.28) |
| **at 4 hrs.** | 239.8 (659.59) | 60.3 (37.04) | 320.8 (983.32) | 28.5 (95.92) | **12.6 (19.02)** | **17.7 (20.09)** |
| **at 8 hrs.** | 63.9 (30.63) | 148.3 (202.45) | 69.4 (88.68) | 51.0 (70.41) | 1.4 (15.13) | 5.6 (25.88) |
| **at 24 hrs.** | 194.9 (472.93) | 78.7 (95.04) | 572.5 (1786.19) | 4.0 (52.90) | -16.6 (19.50) | -1.8 (29.55) |

**Table 5**. Relative change in MRI signal intensity (SI%) and signal intensity from liver imaging at 1, 4, 8 and 24 hours post-dose for fasting subjects (T1) compared to the same subjects after receiving a snack (T22). The SI% obtained from imaging of the subject after the snack is surprisingly at least as good as after fasting.

## Conclusions

The present study supports the existing hypothesis that consumption of a full meal according to Table 1 prior to dosing of an oral manganese based MRI agent, such as Orviglance, significantly retards the imaging quality from subsequent liver MRI. Surprisingly, the present study also supports that subjects having consumed a meal with a calorie

content of about 500 kcal or below (a snack) are still able to undergo MRI using Orviglance with an imaging quality being at least as high as for fasting subjects. These findings significantly support the diagnostic potential of Orviglance, in particular for subjects where fasting can be inconvenient or problematic.

## Claims

1. An MRI composition comprising, separately or together, manganese (II) chloride tetrahydrate, L-alanine, and a vitamin D; wherein the MRI composition is for use in the diagnosis in vivo of liver metastases and/or primary liver cancer in a subject; wherein the subject has consumed a meal from 0 to 8 hours prior to or up to 2 hours subsequent to administration of the MRI composition and wherein the calorie content of the meal is about 500 kcal or below.

2. The MRI composition for use according to claim 1, wherein the composition further comprises one or more water-soluble excipients.

3. The MRI composition for use according to any one of the preceding claims, wherein the subject has reduced nausea, and/or diarrhea, and/or headaches as compared to a fasting subject having been administered said MRI composition.

4. The MRI composition for use according to any one of the preceding claims, wherein the subject has not been fasting for 3 or more hours prior to the administration of the MRI composition.

5. The MRI composition for use according to any one of the preceding claims, wherein the subject suffers from a disease where not eating for more than 8 hours imposes a health risk to the subject and/or the subject has one or more of:

   a. diabetes, such as diabetes type-I or diabetes type-II;
   b. a Body Mass Index (BMI) of 20 or below;
   c. an age of 60 years or above; and/or
   d. hyperthyroidism.

6. The MRI composition for use according to any one of the preceding claims, wherein the subject has consumed the meal from 0 to 7 hours prior to administration of the MRI composition, such as within about 30 minutes, such as within about 45 minutes, such as from 0 to 1 hour, such as from 1 to 2 hours, such as from 2 to 3 hours, such as from 3 to 4 hours, such as from 4 to 5 hours, such as from 5 to 6 hours, such as from 6 to 7 hours prior to administration of the MRI composition.

7. The MRI composition for use according to any one of the preceding claims, wherein the meal comprises a predefined amount of carbohydrates, a predefined amount of fats, and a predefined amount of proteins.

8. The MRI composition for use according to any one of the preceding claims, wherein the predefined amount of carbohydrates in the meal is from 20 gram to 50 gram, such as from 20 to 22 gram, such as from 22 to 24 gram, such as from 24 to 26 gram, such as from 26 to 28 gram, such as from 28 to 30 gram, such as from 30 to 32 gram, such as from 32 to 34 gram, such as from 34 to 36 gram, such as from 36 to 38 gram, such as from 38 to 40 gram, such as from 40 to 42 gram, such as from 42 to 44 gram, such as from 44 to 46 gram, such as from 46 to 48 gram, such as from 48 to 50 gram, and/or wherein the predefined amount of carbohydrates in the meal is from 60 to 200 kcal, such as from 60 to 70 kcal, such as from 70 to 80 kcal, such as from 80 to 90 kcal, such as from 90 to 100 kcal, such as from 100 to 110 kcal, such as from 110 to 120 kcal, such as from 120 to 130 kcal, such as from 130 to 140 kcal, such as from 140 to 150 kcal, such as from 150 to 160 kcal, such as from 160 to 170 kcal, such as from 170 to 180 kcal, such as from 180 to 190 kcal, such as from 190 to 200 kcal, and/or wherein the predefined amount of carbohydrates in the meal is from 50 to 70 kcal% of the total amount of calories in the meal, such as from 50 to 52 kcal%, such as from 52 to 54 kcal%, such as from 54 to 56 kcal%, such as from 56 to 58 kcal%, such as from 58 to 60 kcal%, such as from 60 to 62 kcal%, such as from 62 to 64 kcal%, such as from 64 to 66 kcal%, such as from 66 to 68 kcal%, such as from 68 to 70 kcal% of the total amount of calories in the meal.

9. The MRI composition for use according to any one of the preceding claims, wherein the predefined amount of fats in the meal is from 5 gram to 30 gram, such as from 5 to 10 gram, such as from 10 to 15 gram, such as from 15 to 20 gram, such as from 20 to 25 gram, such as from 25 to 30 gram, and/or wherein the predefined amount of fats in the meal is from 30 to 150 kcal, such as from 30 to 40 kcal, such as from 40 to 50 kcal, such as from 50 to 60 kcal, such as from 60 to 70 kcal, such as from 70 to 80 kcal, such as from 80 to 90 kcal, such as from 90 to 100 kcal, such as from 100 to 110 kcal,

such as from 110 to 120 kcal, such as from 120 to 130 kcal, such as from 130 to 140 kcal, such as from 140 to 150 kcal, and/or wherein the predefined amount of fats in the meal is from 25 to 45 kcal% of the total amount of calories in the meal, such as from 25 to 27 kcal%, such as from 27 to 29 kcal%, such as from 29 to 31 kcal%, such as from 31 to 33 kcal%, such as from 33 to 35 kcal%, such as from 35 to 37 kcal%, such as from 37 to 39 kcal%, such as from 39 to 41 kcal%, such as from 41 to 43 kcal%, such as from 43 to 45 kcal% of the total amount of calories in the meal.

**10.** The MRI composition for use according to any one of the preceding claims, wherein the predefined amount of proteins in the meal is from 0 gram to 10 gram, such as from 0 to 2 gram, such as from 2 to 4 gram, such as from 4 to 6 gram, such as from 6 to 8 gram, such as from 8 to 10 gram, and/or wherein the predefined amount of proteins in the meal is from 0 to 50 kcal, such as from 0 to 5 kcal, such as from 5 to 10 kcal, such as from 10 to 15 kcal, such as from 15 to 20 kcal, such as from 20 to 25 kcal, such as from 25 to 30 kcal, such as from 30 to 35 kcal, such as from 35 to 40 kcal, such as from 40 to 45 kcal, such as from 45 to 50 kcal, and/or wherein the predefined amount of proteins in the meal is from 0 to 10 kcal% of the total amount of calories in the meal, such as from 0 to 1 kcal%, such as from 1 to 2 kcal%, such as from 2 to 3 kcal%, such as from 3 to 4 kcal%, such as from 4 to 5 kcal%, such as from 5 to 6 kcal%, such as from 6 to 7 kcal%, such as from 7 to 8 kcal%, such as from 8 to 9 kcal%, such as from 9 to 10 kcal% of the total amount of calories in the meal.

**11.** The MRI composition for use according to any one of the preceding claims, wherein the meal comprises one or more food items selected from the group consisting of: bread, such as toast bread, and fruit juice, such as lime juice and/or wherein the juice is provided at a volume of from 100 mL to 400 mL.

**12.** The MRI composition for use according to any one of the preceding claims, wherein the calorie content of the meal is from 100 kcal to 500 kcal, such as from 100 to 110 kcal, such as from 110 kcal to 120 kcal, such as from 120 kcal to 130 kcal, such as from 130 kcal to 140 kcal, such as from 140 kcal to 150 kcal, such as from 150 kcal to 160 kcal, such as from 160 kcal to 170 kcal, such as from 170 kcal to 180 kcal, such as from 180 kcal to 190 kcal, such as from 190 kcal to 200 kcal, such as from 200 kcal to 210 kcal, such as from 210 kcal to 220 kcal, such as from 220 kcal to 230 kcal, such as from 230 kcal to 240 kcal, such as from 240 kcal to 250 kcal, such as from 250 kcal to 260 kcal, such as from 260 kcal to 270 kcal, such as 270 kcal to 280 kcal, such as from 280 kcal to 290 kcal, such as from 290 kcal to 300 kcal, such as from 300 kcal to 310 kcal, such as from 310 kcal to 320 kcal, such as from 320 kcal to 330 kcal, such as from 330 kcal to 340 kcal, such as from 340 kcal to 350 kcal, such as from 350 kcal to 360 kcal, such as from 360 kcal to 370 kcal, such as from 370 kcal to 380 kcal, such as from 380 kcal to 390 kcal, such as from 390 kcal to 400 kcal.

**13.** A method for Magnetic Resonance Imaging (MRI) of a subject comprising, administering a composition comprising separately or together, manganese (II) chloride tetrahydrate, L-alanine and a vitamin D, and optionally one or more water-soluble excipients to the subject; and performing MRI on said subject; wherein the subject has consumed a meal from 0 to 8 hours such as from 0 to 1 hour, such as from 1 to 2 hours, such as from 2 to 3 hours, such as from 3 to 4 hours, such as from 4 to 5 hours, such as from 5 to 6 hours prior to or up to 2 hours subsequent to administration of the MRI composition, and wherein the calorie content of the meal is about 500 kcal or below.

**14.** A meal for use in a method of reducing or preventing nausea, diarrhea, and/or headaches in a subject receiving an MRI composition comprising, separately or together, manganese (II) chloride tetrahydrate, L-alanine and a vitamin D, and optionally one or more water-soluble excipients; the method comprising:

i) administering the meal to the subject; and
ii) administering the MRI composition to the subject;

wherein the meal is administered from 0 to 8 hours such as from 0 to 1 hour, such as from 1 to 2 hours, such as from 2 to 3 hours, such as from 3 to 4 hours, such as from 4 to 5 hours, such as from 5 to 6 hours prior to or up to 2 hours subsequent to administering the MRI composition, further wherein the calorie content of the meal is about 500 kcal or below.

**15.** The method according to claim 13 or the meal for use according to claim 14,
wherein the imaging quality is unaffected compared to a method where the MRI composition is administered to a fasting subject, wherein the fasting subject has not been eating for more than 8 hours.

## Patentansprüche

**1.** MRI-Zusammensetzung, umfassend, getrennt oder zusammen, Mangan(II)-chlorid-Tetrahydrat, L-Alanin und Vita-

min D; wobei die MRI-Zusammensetzung zur Verwendung bei der Diagnose in vivo von Lebermetastasen und/oder primärem Leberkrebs bei einem Subjekt vorgesehen ist; wobei das Subjekt eine Mahlzeit von 0 bis 8 Stunden vor oder bis zu 2 Stunden nach der Verabreichung der MRI-Zusammensetzung zu sich genommen hat und wobei der Kaloriengehalt der Mahlzeit etwa 500 kcal oder weniger beträgt.

**2.** MRI-Zusammensetzung zur Verwendung nach Anspruch 1, wobei die Zusammensetzung ferner einen oder mehrere wasserlösliche Hilfsstoffe umfasst.

**3.** MRI-Zusammensetzung zur Verwendung nach einem der vorstehenden Ansprüche, wobei das Subjekt im Vergleich zu einem nüchternen Subjekt, dem die genannte MRI-Zusammensetzung verabreicht wurde, verringerte Übelkeit und/oder Durchfall und/oder Kopfschmerzen aufweist.

**4.** MRI-Zusammensetzung zur Verwendung nach einem der vorstehenden Ansprüche, wobei das Subjekt vor der Verabreichung der MRI-Zusammensetzung nicht 3 oder mehr Stunden gefastet hat.

**5.** MRI-Zusammensetzung zur Verwendung nach einem der vorstehenden Ansprüche, wobei das Subjekt an einer Erkrankung leidet, bei der Nichtessen für mehr als 8 Stunden ein Gesundheitsrisiko für das Subjekt darstellt und/oder das Subjekt eines oder mehrere der folgenden Merkmale aufweist:

a. Diabetes, wie Diabetes Typ-I oder Diabetes Typ-II;
b. einen Body-Mass-Index (BMI) von 20 oder weniger;
c. ein Alter von 60 Jahren oder mehr; und/oder
d. Hyperthyreose.

**6.** MRI-Zusammensetzung zur Verwendung nach einem der vorstehenden Ansprüche, wobei das Subjekt die Mahlzeit 0 bis 7 Stunden vor der Verabreichung der MRI-Zusammensetzung zu sich genommen hat, wie innerhalb von etwa 30 Minuten, wie innerhalb von etwa 45 Minuten, wie von 0 bis 1 Stunde, wie von 1 bis 2 Stunden, wie von 2 bis 3 Stunden, wie von 3 bis 4 Stunden, wie von 4 bis 5 Stunden, wie von 5 bis 6 Stunden, wie von 6 bis 7 Stunden vor der Verabreichung der MRI-Zusammensetzung.

**7.** MRI-Zusammensetzung zur Verwendung nach einem der vorstehenden Ansprüche, wobei die Mahlzeit eine vorbestimmte Menge an Kohlenhydraten, eine vorbestimmte Menge an Fetten und eine vorbestimmte Menge an Proteinen umfasst.

**8.** MRI-Zusammensetzung zur Verwendung nach einem der vorstehenden Ansprüche, wobei die vorbestimmte Menge an Kohlenhydraten in der Mahlzeit 20 Gramm bis 50 Gramm beträgt, wie von 20 bis 22 Gramm, wie von 22 bis 24 Gramm, wie von 24 bis 26 Gramm, wie von 26 bis 28 Gramm, wie von 28 bis 30 Gramm, wie von 30 bis 32 Gramm, wie von 32 bis 34 Gramm, wie von 34 bis 36 Gramm, wie von 36 bis 38 Gramm, wie von 38 bis 40 Gramm, wie von 40 bis 42 Gramm, wie von 42 bis 44 Gramm, wie von 44 bis 46 Gramm, wie von 46 bis 48 Gramm, wie von 48 bis 50 Gramm, und/oder wobei die vorbestimmte Menge an Kohlenhydraten in der Mahlzeit 60 bis 200 kcal beträgt, wie von 60 bis 70 kcal, wie von 70 bis 80 kcal, wie von 80 bis 90 kcal, wie von 90 bis 100 kcal, wie von 100 bis 110 kcal, wie von 110 bis 120 kcal, wie von 120 bis 130 kcal, wie von 130 bis 140 kcal, wie von 140 bis 150 kcal, wie von 150 bis 160 kcal, wie von 160 bis 170 kcal, wie von 170 bis 180 kcal, wie von 180 bis 190 kcal, wie von 190 bis 200 kcal, und/oder wobei die vorbestimmte Menge an Kohlenhydraten in der Mahlzeit 50 bis 70 kcal% der Gesamtmenge der Kalorien in der Mahlzeit beträgt, wie von 50 bis 52 kcal%, wie von 52 bis 54 kcal%, wie von 54 bis 56 kcal%, wie von 56 bis 58 kcal%, wie von 58 bis 60 kcal%, wie von 60 bis 62 kcal%, wie von 62 bis 64 kcal%, wie von 64 bis 66 kcal%, wie von 66 bis 68 kcal%, wie von 68 bis 70 kcal% der Gesamtmenge der Kalorien in der Mahlzeit.

**9.** MRI-Zusammensetzung zur Verwendung nach einem der vorstehenden Ansprüche, wobei die vorbestimmte Menge an Fetten in der Mahlzeit 5 Gramm bis 30 Gramm beträgt, wie von 5 bis 10 Gramm, wie von 10 bis 15 Gramm, wie von 15 bis 20 Gramm, wie von 20 bis 25 Gramm, wie von 25 bis 30 Gramm, und/oder wobei die vorbestimmte Menge an Fetten in der Mahlzeit 30 bis 150 kcal beträgt, wie von 30 bis 40 kcal, wie von 40 bis 50 kcal, wie von 50 bis 60 kcal, wie von 60 bis 70 kcal, wie von 70 bis 80 kcal, wie von 80 bis 90 kcal, wie von 90 bis 100 kcal, wie von 100 bis 110 kcal, wie von 110 bis 120 kcal, wie von 120 bis 130 kcal, wie von 130 bis 140 kcal, wie von 140 bis 150 kcal, und/oder wobei die vorbestimmte Menge an Fetten in der Mahlzeit 25 bis 45 kcal% der Gesamtmenge der Kalorien in der Mahlzeit beträgt, wie von 25 bis 27 kcal%, wie von 27 bis 29 kcal%, wie von 29 bis 31 kcal%, wie von 31 bis 33 kcal%, wie von 33 bis 35 kcal%, wie von 35 bis 37 kcal%, wie von 37 bis 39 kcal%, wie von 39 bis 41 kcal%, wie von 41 bis 43 kcal%, wie von 43 bis 45 kcal% der Gesamtmenge der Kalorien in der Mahlzeit.

10. MRI-Zusammensetzung zur Verwendung nach einem der vorstehenden Ansprüche, wobei die vorbestimmte Menge an Proteinen in der Mahlzeit 0 Gramm bis 10 Gramm beträgt, wie von 0 bis 2 Gramm, wie von 2 bis 4 Gramm, wie von 4 bis 6 Gramm, wie von 6 bis 8 Gramm, wie von 8 bis 10 Gramm, und/oder wobei die vorbestimmte Menge an Proteinen in der Mahlzeit 0 bis 50 kcal beträgt, wie von 0 bis 5 kcal, wie von 5 bis 10 kcal, wie von 10 bis 15 kcal, wie von 15 bis 20 kcal, wie von 20 bis 25 kcal, wie von 25 bis 30 kcal, wie von 30 bis 35 kcal, wie von 35 bis 40 kcal, wie von 40 bis 45 kcal, wie von 45 bis 50 kcal, und/oder wobei die vorbestimmte Menge an Proteinen in der Mahlzeit 0 bis 10 kcal% der Gesamtmenge der Kalorien in der Mahlzeit beträgt, wie von 0 bis 1 kcal%, wie von 1 bis 2 kcal%, wie von 2 bis 3 kcal%, wie von 3 bis 4 kcal%, wie von 4 bis 5 kcal%, wie von 5 bis 6 kcal%, wie von 6 bis 7 kcal%, wie von 7 bis 8 kcal%, wie von 8 bis 9 kcal%, wie von 9 bis 10 kcal% der Gesamtmenge der Kalorien in der Mahlzeit.

11. MRI-Zusammensetzung zur Verwendung nach einem der vorstehenden Ansprüche, wobei die Mahlzeit aus einem oder mehreren Lebensmittel aus der Gruppe bestehend aus: Brot, wie Toastbrot, und Fruchtsaft, wie Limettensaft, ausgewählt ist und/oder wobei der Saft in einem Volumen von 100 mL bis 400 mL bereitgestellt wird.

12. MRI-Zusammensetzung zur Verwendung nach einem der vorstehenden Ansprüche, wobei der Kaloriengehalt der Mahlzeit 100 kcal bis 500 kcal beträgt, wie von 100 bis 110 kcal, wie von 110 kcal bis 120 kcal, wie von 120 kcal bis 130 kcal, wie von 130 kcal bis 140 kcal, wie von 140 kcal bis 150 kcal, wie von 150 kcal bis 160 kcal, wie von 160 kcal bis 170 kcal, wie von 170 kcal bis 180 kcal, wie von 180 kcal bis 190 kcal, wie von 190 kcal bis 200 kcal, wie von 200 kcal bis 210 kcal, wie von 210 kcal bis 220 kcal, wie von 220 kcal bis 230 kcal, wie von 230 kcal bis 240 kcal, wie von 240 kcal bis 250 kcal, wie von 250 kcal bis 260 kcal, wie von 260 kcal bis 270 kcal, wie von 270 kcal bis 280 kcal, wie von 280 kcal bis 290 kcal, wie von 290 kcal bis 300 kcal, wie von 300 kcal bis 310 kcal, wie von 310 kcal bis 320 kcal, wie von 320 kcal bis 330 kcal, wie von 330 kcal bis 340 kcal, wie von 340 kcal bis 350 kcal, wie von 350 kcal bis 360 kcal, wie von 360 kcal bis 370 kcal, wie von 370 kcal bis 380 kcal, wie von 380 kcal bis 390 kcal, wie von 390 kcal bis 400 kcal.

13. Verfahren zur Magnetresonanztomographie (MRI) eines Subjekts, umfassend das Verabreichen einer Zusammensetzung an das Subjekt, umfassend getrennt oder zusammen Mangan(II)-chlorid-Tetrahydrat, L-Alanin und Vitamin D sowie optional einen oder mehrere wasserlösliche Hilfsstoffe; und das Durchführen der MRI an dem genannten Subjekt; wobei das Subjekt eine Mahlzeit von 0 bis 8 Stunden, beispielsweise von 0 bis 1 Stunde, von 1 bis 2 Stunden, von 2 bis 3 Stunden, von 3 bis 4 Stunden, von 4 bis 5 Stunden oder von 5 bis 6 Stunden vor oder bis zu 2 Stunden nach der Verabreichung der MRI-Zusammensetzung zu sich genommen hat, und wobei der Kaloriengehalt der Mahlzeit etwa 500 kcal oder weniger beträgt.

14. Mahlzeit zur Verwendung in einem Verfahren zur Verringerung oder Verhinderung von Übelkeit, Durchfall und/oder Kopfschmerzen bei einem Subjekt, das eine MRI-Zusammensetzung erhält, umfassend getrennt oder zusammen Mangan(II)-chlorid-Tetrahydrat, L-Alanin und ein Vitamin D und optional einen oder mehrere wasserlösliche Hilfsstoffe; wobei das Verfahren umfasst:

    i) Verabreichen der Mahlzeit an das Subjekt; und
    ii) Verabreichen der MRI-Zusammensetzung an das Subjekt;

    wobei die Mahlzeit von 0 bis 8 Stunden, beispielsweise von 0 bis 1 Stunde, von 1 bis 2 Stunden, von 2 bis 3 Stunden, von 3 bis 4 Stunden, von 4 bis 5 Stunden oder von 5 bis 6 Stunden vor oder bis zu 2 Stunden nach dem Verabreichen der MRI-Zusammensetzung eingenommen wird und wobei der Kaloriengehalt der Mahlzeit etwa 500 kcal oder weniger beträgt.

15. Verfahren nach Anspruch 13 oder Mahlzeit zur Verwendung nach Anspruch 14, wobei die Bildgebungsqualität im Vergleich zu einem Verfahren unbeeinträchtigt ist, bei dem die MRI-Zusammensetzung einem nüchternen Subjekt verabreicht wird, wobei das nüchterne Subjekt länger als 8 Stunden nichts gegessen hat.

## Revendications

1. Composition d'IRM comprenant, séparément ou ensemble, du chlorure de manganèse (II) tétrahydraté, de la L-alanine et de la vitamine D ; dans laquelle la composition d'IRM est pour une utilisation dans le diagnostic in vivo des métastases hépatiques et/ou du cancer primitif du foie chez un sujet ; dans laquelle le sujet a consommé un repas entre 0 et 8 heures avant ou jusqu'à 2 heures après l'administration de la composition d'IRM et dans laquelle la teneur en calories du repas est d'environ 500 kcal ou moins.

2. Composition d'IRM pour une utilisation selon la revendication 1, dans laquelle la composition comprend également un ou plusieurs excipients hydrosolubles.

3. Composition d'IRM pour une utilisation selon l'une quelconque des revendications précédentes, dans laquelle le sujet présente une réduction de nausée, et/ou de diarrhée, et/ou de maux de tête par rapport à un sujet à jeun ayant reçu ladite composition d'IRM.

4. Composition d'IRM pour une utilisation selon l'une quelconque des revendications précédentes, dans laquelle le sujet n'a pas été à jeun pendant 3 heures ou plus avant l'administration de la composition d'IRM.

5. Composition d'IRM pour une utilisation selon l'une quelconque des revendications précédentes, dans laquelle le sujet souffre d'une maladie pour laquelle le fait de ne pas s'alimenter pendant plus de 8 heures constitue un risque pour la santé du sujet et/ou le sujet présente un ou plusieurs des éléments suivants :

a. diabète, tel que diabète de type I ou diabète de type II ;
b. un indice de masse corporelle (IMC) de 20 ou moins ;
c. un âge de 60 ans ou plus ; et/ou
d. hyperthyroïdie.

6. Composition d'IRM pour une utilisation selon l'une quelconque des revendications précédentes, dans laquelle le sujet a consommé le repas entre 0 et 7 heures avant l'administration de la composition d'IRM, par exemple à environ 30 minutes, par exemple à environ 45 minutes, par exemple entre 0 et 1 heure, par exemple entre 1 et 2 heures, par exemple entre 2 et 3 heures, par exemple entre 3 et 4 heures, par exemple entre 4 et 5 heures, par exemple entre 5 et 6 heures, par exemple entre 6 et 7 heures avant l'administration de la composition d'IRM.

7. Composition d'IRM pour une utilisation selon l'une quelconque des revendications précédentes, dans laquelle le repas comprend une quantité prédéfinie de glucides, une quantité prédéfinie de lipides et une quantité prédéfinie de protéines.

8. Composition d'IRM pour une utilisation selon l'une quelconque des revendications précédentes, dans laquelle la quantité prédéfinie de glucides dans le repas est comprise entre 20 grammes et 50 grammes, par exemple entre 20 et 22 grammes, par exemple entre 22 et 24 grammes, par exemple entre 24 et 26 grammes, par exemple entre 26 et 28 grammes, par exemple entre 28 et 30 grammes, par exemple entre 30 et 32 grammes, par exemple entre 32 et 34 grammes, par exemple entre 34 et 36 grammes, par exemple entre 36 et 38 grammes, par exemple entre 38 et 40 grammes, par exemple entre 40 et 42 grammes, par exemple entre 42 et 44 grammes, par exemple entre 44 et 46 grammes, par exemple entre 46 et 48 grammes, par exemple entre 48 et 50 grammes, et/ou dans laquelle la quantité prédéfinie de glucides dans le repas est comprise entre 60 et 200 kcal, par exemple entre 60 et 70 kcal, par exemple entre 70 et 80 kcal, par exemple entre 80 et 90 kcal, par exemple entre 90 et 100 kcal, par exemple entre 100 et 110 kcal, par exemple entre 110 et 120 kcal, par exemple entre 120 et 130 kcal, par exemple entre 130 et 140 kcal, par exemple entre 140 et 150 kcal, par exemple entre 150 et 160 kcal, par exemple entre 160 et 170 kcal, par exemple entre 170 et 180 kcal, par exemple entre 180 et 190 kcal, par exemple entre 190 et 200 kcal, et/ou dans laquelle la quantité prédéfinie de glucides dans le repas est comprise entre 50 et 70 kcal % de la quantité totale de calories dans le repas, par exemple entre 50 et 52 kcal %, par exemple entre 52 et 54 kcal %, par exemple entre 54 et 56 kcal %, par exemple entre 56 et 58 kcal %, par exemple entre 58 et 60 kcal %, par exemple entre 60 et 62 kcal %, par exemple entre 62 et 64 kcal %, par exemple entre 64 et 66 kcal %, par exemple entre 66 et 68 kcal %, par exemple entre 68 et 70 kcal % de la quantité totale de calories dans le repas.

9. Composition d'IRM pour une utilisation selon l'une quelconque des revendications précédentes, dans laquelle la quantité prédéfinie de lipides dans le repas est comprise entre 5 grammes et 30 grammes, par exemple entre 5 et 10 grammes, par exemple entre 10 et 15 grammes, par exemple entre 15 et 20 grammes, par exemple entre 20 et 25 grammes, par exemple entre 25 et 30 grammes, et/ou dans laquelle la quantité prédéfinie de lipides dans le repas est comprise entre 30 et 150 kcal, par exemple entre 30 et 40 kcal, par exemple entre 40 et 50 kcal, par exemple entre 50 et 60 kcal, par exemple entre 60 et 70 kcal, par exemple entre 70 et 80 kcal, par exemple entre 80 et 90 kcal, par exemple entre 90 et 100 kcal, par exemple entre 100 et 110 kcal, par exemple entre 110 et 120 kcal, par exemple entre 120 et 130 kcal, par exemple entre 130 et 140 kcal, par exemple entre 140 et 150 kcal, et/ou dans laquelle la quantité prédéfinie de lipides dans le repas est comprise entre 25 et 45 kcal % de la quantité totale de calories dans le repas, par exemple entre 25 et 27 kcal %, par exemple entre 27 et 29 kcal %, par exemple entre 29 et 31 kcal %, par exemple entre 31 et 33 kcal %, par exemple entre 33 et 35 kcal %, par exemple entre 35 et 37 kcal %, par exemple entre 37 et 39

kcal %, par exemple entre 39 et 41 kcal %, par exemple entre 41 et 43 kcal %, par exemple entre 43 et 45 kcal % de la quantité totale de calories dans le repas.

**10.** Composition d'IRM pour une utilisation selon l'une quelconque des revendications précédentes, dans laquelle la quantité prédéfinie de protéines dans le repas est comprise entre 0 gramme et 10 grammes, par exemple entre 0 et 2 grammes, par exemple entre 2 et 4 grammes, par exemple entre 4 et 6 grammes, par exemple entre 6 et 8 grammes, par exemple entre 8 et 10 grammes, et/ou dans laquelle la quantité prédéfinie de protéines dans le repas est comprise entre 0 et 50 kcal, par exemple entre 0 et 5 kcal, par exemple entre 5 et 10 kcal, par exemple entre 10 et 15 kcal, par exemple entre 15 et 20 kcal, par exemple entre 20 et 25 kcal, par exemple entre 25 et 30 kcal, par exemple entre 30 et 35 kcal, par exemple entre 35 et 40 kcal, par exemple entre 40 et 45 kcal, par exemple entre 45 et 50 kcal, et/ou dans laquelle la quantité prédéfinie de protéines dans le repas est comprise entre 0 et 10 kcal % de la quantité totale de calories dans le repas, par exemple entre 0 et 1 kcal %, par exemple entre 1 et 2 kcal %, par exemple entre 2 et 3 kcal %, par exemple entre 3 et 4 kcal %, par exemple entre 4 et 5 kcal %, par exemple entre 5 et 6 kcal %, par exemple entre 6 et 7 kcal %, par exemple entre 7 et 8 kcal %, par exemple entre 8 et 9 kcal %, par exemple entre 9 et 10 kcal % de la quantité totale de calories dans le repas.

**11.** Composition d'IRM pour une utilisation selon l'une quelconque des revendications précédentes, dans laquelle le repas comprend un ou plusieurs aliments choisis dans le groupe constitué par : du pain, tel que du pain grillé, et du jus de fruit, tel que du jus de citron vert et/ou dans laquelle le jus est fourni à un volume de 100 mL à 400 mL.

**12.** Composition d'IRM pour une utilisation selon l'une quelconque des revendications précédentes, dans laquelle la teneur en calories du repas est comprise entre 100 kcal et 500 kcal, par exemple entre 100 et 110 kcal, par exemple entre 110 et 120 kcal, par exemple entre 120 et 130 kcal, par exemple entre 130 et 140 kcal, par exemple entre 140 et 150 kcal, par exemple entre 150 et 160 kcal, par exemple entre 160 et 170 kcal, par exemple entre 170 et 180 kcal, par exemple entre 180 et 190 kcal, par exemple entre 190 et 200 kcal, par exemple entre 200 et 210 kcal, par exemple entre 210 et 220 kcal, par exemple entre 220 et 230 kcal, par exemple entre 230 et 240 kcal, par exemple entre 240 et 250 kcal, par exemple entre 250 et 260 kcal, par exemple entre 260 et 270 kcal, par exemple entre 270 et 280 kcal, par exemple entre 280 et 290 kcal, par exemple entre 290 et 300 kcal, par exemple entre 300 et 310 kcal, par exemple entre 310 et 320 kcal, par exemple entre 320 et 330 kcal, par exemple entre 330 et 340 kcal, par exemple entre 340 et 350 kcal, par exemple entre 350 et 360 kcal, par exemple entre 360 et 370 kcal, par exemple entre 370 et 380 kcal, par exemple entre 380 et 390 kcal, par exemple entre 390 et 400 kcal.

**13.** Procédé d'imagerie par résonance magnétique (IRM) d'un sujet comprenant l'administration au sujet d'une composition comprenant, séparément ou ensemble, du chlorure de manganèse (II) tétrahydraté, de la L-alanine et de la vitamine D, et éventuellement un ou plusieurs excipients hydrosolubles ; et la réalisation d'une IRM sur ledit sujet ; dans lequel le sujet a consommé un repas entre 0 et 8 heures, par exemple entre 0 et 1 heure, par exemple entre 1 et 2 heures, par exemple entre 2 et 3 heures, par exemple entre 3 et 4 heures, par exemple entre 4 et 5 heures, par exemple entre 5 et 6 heures avant ou jusqu'à 2 heures après l'administration de la composition d'IRM, et dans lequel la teneur en calories du repas est d'environ 500 kcal ou moins.

**14.** Repas pour une utilisation dans un procédé de réduction ou de prévention des nausées, de la diarrhée et/ou des maux de tête chez un sujet recevant une composition d'IRM comprenant, séparément ou ensemble, du chlorure de manganèse (II) tétrahydraté, de la L-alanine et de la vitamine D, et éventuellement un ou plusieurs excipients hydrosolubles ; le procédé comprenant :

i) l'administration du repas au sujet ; et
ii) l'administration de la composition d'IRM au sujet ;

dans lequel le repas est administré entre 0 et 8 heures, par exemple entre 0 et 1 heure, par exemple entre 1 et 2 heures, par exemple entre 2 et 3 heures, par exemple entre 3 et 4 heures, par exemple entre 4 et 5 heures, par exemple entre 5 et 6 heures avant ou jusqu'à 2 heures après l'administration de la composition d'IRM et dans laquelle en outre la teneur en calories du repas est d'environ 500 kcal ou moins.

**15.** Procédé selon la revendication 13 ou repas pour une utilisation selon la revendication 14, dans lequel la qualité d'imagerie n'est pas affectée par rapport à un procédé dans lequel la composition d'IRM est administrée à un sujet à jeun, dans lequel le sujet à jeun n'a pas mangé depuis plus de 8 heures.

Study Specific Screening
Period-1 Fasting
24 subjects for 800mg in 200mL drinking water
Period-2 Fed after 7 days washout
12 subjects after a full meal for 800mg in 200mL drinking water
12 subjects after a snack for 800mg in 200mL drinking water
MRI of the liver will be carried out at 4 hour prior to administration of dose (within 60 minutes) and post-dose at 1 ± 0.5, 4 ± 0.5, 8 ± 0.5, and 24 ± 0.5 hours in each period.

Fig. 1

Fasting vs. Full Meal
20
10
0
-10
-20
-30
Liver SI%
0
4
8
12
16
20
24
Time (h)
Fasting
Full meal

Fig. 2

Fasting vs. Snack
20
10
0
-10
-20
-30
Liver SI%
0
4
8
12
16
20
24
Time (h)
Fasting
Snack

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9605867 A

### Non-patent literature cited in the description

- **CHABANOVA et al.** *Academic Radiology*, 2006, vol. 13 (7)
- **LEANDER et al.** *Investigative Radiology*, 2010, vol. 45